**Europäisches Patentamt**

**European Patent Office**

**Office européen des brevets**

(19)

(11) Numéro de publication: **0 128 054 B1**

(12) # FASCICULE DE BREVET EUROPEEN

(45) Date de publication du fascicule du brevet:
**28.12.88**

(51) Int. Cl.⁴: **G 06 F 15/42**

(21) Numéro de dépôt: **84400725.2**

(22) Date de dépôt: **11.04.84**

(54) **Appareil portatif de saisie et de traitement d'informations relatives à la diététique et/ou à la santé d'une personne.**

(30) Priorité: **12.04.83 FR 8305949**

(43) Date de publication de la demande:
**12.12.84 Bulletin 84/50**

(45) Mention de la délivrance du brevet:
**28.12.88 Bulletin 88/52**

(84) Etats contractants désignés:
**AT BE CH DE FR GB IT LI LU NL SE**

(56) Documents cités:
**US-A- 4 281 330**
**US-A- 4 321 674**

(73) Titulaire: **Blum, André, 26, Chemin du Grand Buisson, F-25000 Besancon (FR)**
Titulaire: **Blum, Dominique, 14, rue du Vallon, Thise, Roche lez Beaupre (FR)**

(72) Inventeur: **Blum, Dominique, 14, rue du Vallon Thise, F-25220 Roche lez Beaupre (FR)**
Inventeur: **Grandmottet, Pierre, 6, rue des Vieilles Perrières, F-25000 Besancon (FR)**
Inventeur: **Bechtel, Pierre, 32, rue de Dôle, F-25000 Besancon (FR)**

(74) Mandataire: **Wagret, Jean-Michel et al, Propi Conseils 23 rue de Léningrad, F-75008 Paris (FR)**

**Description**

L'invention est relative à un appareil portatif de saisie et de traitement d'informations relatives à la diététique et/ou à la santé d'une personne.

L'invention concerne plus particulièrement, mais non exclusivement, les personnes soumises à un contrôle de leur régime alimentaire ou les personnes soumises à un traitement médical, telles que les diabétiques.

L'invention a pour but, surtout, de fournir un appareil qui permette aux personnes concernées de suivre plus facilement les prescriptions diététiques et/ou médicales, de prendre en compte des informations répétitives et de pouvoir communiquer celles-ci au médecin traitant ou à un organisme de surveillance.

On connaît, par le brevet des Etats-Unis No 4 321 674, un dispositif apte à accumuler et afficher le nombre de calories correspondant à la nourriture et/ou à la boisson prise par l'utilisateur. Ce dispositif connu comporte:

– une série de touches pour l'entrée d'informations relatives à la diététique et/ou à la santé de l'usager,

– au moins une mémoire morte dans laquelle sont stockées des données et des instructions en relation avec ces informations,

– des moyens d'affichage des renseignements en provenance de la mémoire morte et concernant les informations introduites,

– des moyens d'entrée effective et de refus d'entrée de ces informations,

– des moyens de calcul propres à déterminer, à partir des informations entrées et des données stockées dans la mémoire morte, des instructions destinées à l'usager de l'appareil et relatives à la diététique et/ou à la santé de l'usager, et

– des moyens d'alimentation électrique principale (batteries) de l'appareil.

Par ailleurs, l'usager dispose d'un tableau des différents types de nourriture lui précisant quelles touches il doit manœuvrer et combien de fois, suivant la nature et la quantité de la nourriture ou boisson.

Il s'agit là d'un dispositif simplifié ne donnant que des valeurs approximatives des teneurs en hydrates de carbone, protéines et graisses et en calories des repas, le nombre des types d'aliments et/ou de boissons étant fort réduit; ce dispositif nécessite que l'usager se réfère à un tableau donnant la composition des aliments, ce qui risque d'entraîner des erreurs de détermination de la touche à manipuler et du nombre de fois qu'il faut la manipuler pour tenir compte des quantités.

Par ailleurs, ce dispositif ne permet pas de faire intervenir les horaires des prises de repas, ni de mémoriser la nature et les quantités des différents aliments et boissons pris par l'usager, ni enfin de transférer les informations à une autre unité, telle qu'un ordinateur, permettant un contrôle et une surveillance par un médecin ou un organisme.

Le brevet des Etats-Unis No 4 281 330 décrit un dispositif qui permet de surveiller et enregistrer des traitements médicaux avec des touches pour les différents traitements, les divers médicaments et les quantités de médicaments. Une horloge est prévue. Les traitements, médicaments, quantités et temps sont traités dans un microprocesseur du dispositif, puis transmis pour enregistrement à une imprimante thermique et un enregistreur à bande, ce dernier recevant également les indications d'un électrocardiographe.

On notera que dans ces deux dispositifs connus:

– les touches sont pré-assignées en permanence à un type de nourriture ou de boisson, dans l'un, ou à un traitement, un médicament, une quantité, dans l'autre, sans possibilité d'évolution ou de modification, le premier dispositif étant purement diététique et le second purement médical:

– on ne peut réaliser le transfert direct de la totalité des données introduites avec la date de cette introduction à une autre unité, telle qu'un ordinateur, apte à être utilisée par un médecin ou un organisme de surveillance pour contrôler la diététique et/ou la santé de l'usager et établir des prescriptions pour celui-ci.

La présente invention a pour objet de pallier les inconvénients précités en réalisant un appareil portatif autonome et personnalisable qui permet d'introduire un grand nombre de données très précises concernant l'alimentation et/ou le traitement médical d'une personne, de mémoriser ces données avec le moment de leur introduction et de transférer ces données avec leur moment d'introduction à une autre unité.

A cet effet, l'appareil portatif de saisie et de traitement d'informations relatives à la diététique et/ou à la santé d'une personne, du type comportant, une série de touches alpha-numériques pour l'entrée d'informations relatives à la diététique et/ou à la santé de l'usager; au moins une mémoire morte dans laquelle sont stockées des données et des instructions en relation avec ces informations; des moyens d'affichage des informations introduites et des renseignements les concernant de provenance de la mémoire morte: des moyens d'entrée effective et de refus d'entrée de ces informations; des moyens de calcul propres à déterminer, à partir des informations entrées et des données stockées dans la mémoire morte, des instructions destinées à l'usager de l'appareil et relatives à la diététique et/ou à la santé de l'usager; des moyens d'alimentation électrique principale de l'appareil est caractérisé, selon l'invention, en ce qu'il comporte également des moyens d'horloge permettant de dater chaque entrée d'information; au moins une mémoire vive propre à stocker les informations entrées ainsi que leur date; et des moyens de communication pour permettre le transfert des informations stockées dans la mémoire vive vers une unité de traitement d'informations extérieure, ainsi qu'en retour l'introduction, dans cette mémoire vive, d'instructions en provenance de ladite unité.

Les moyens d'affichage peuvent également servir à l'affichage des instructions contenues dans la mémoire vive.

L'entrée des informations, par frappe sur le clavier, peut se faire à l'aide d'un code chiffré ou d'un code mnémonique; il est alors avantageux de prévoir sur l'appareil lui-même, par exemple au dos de l'appareil, un lexique permettant d'établir la correspondance entre les informations en clair et les chiffres du code. Par exemple, dans le cas d'un régime alimentaire, le lexique donnera la correspondance entre chaque aliment concerné et les chiffres du code.

Lorsque l'appareil est destiné à des personnes soumises à un régime alimentaire, par exemple à des diabétiques, la mémoire morte contient des données relatives à un certain nombre d'aliments, ces données étant notamment constituées par la teneur moyenne en protides, lipides, glucides, alcool et calories de l'aliment en question, et les moyens de calcul sont agencés pour déterminer, pour chaque aliment, en fonction de la quantité qui sera absorbée, la quantité de protides, lipides, glucides, alcool et calories.

Le détail qualitatif et quantitatif du repas est stocké, avec sa date, dans la mémoire vive. Cette mémoire vive peut, en outre, contenir des informations personnalisées qui sont inscrites, par exemple, lors d'une consultation médicale, et qui concernent les quantités de référence quotidiennes de protides, lipides, glucides, alcool, calories qui peuvent être consommées par la personne intéressée, et les moyens de calcul font apparaître, avant chaque entrée d'information relative à un repas ou à une absorption d'aliment, le crédit disponible en protides, lipides, glucides, alcool, calories, pour la journée en cours; ces moyens de calcul modifient, au fur et à mesure, au cours de la journée, le crédit disponible en fonction des informations introduites; ce crédit disponible est remis à sa valeur de référence à une heure déterminée, par exemple à minuit, pour la journée suivante.

Avantageusement, le clavier, pour introduire les informations concernant la diététique et/ou la santé, dans un appareil destiné par exemple à des diabétiques, comporte une touche «repas», une touche «glycémie» et une touche «traitement» et l'appareil est agencé de telle sorte que lorsque l'utilisateur a appuyé sur l'une de ces trois touches, la mise sous tension s'effectue et les informations introduites ultérieurement par l'utilisateur à l'aide des touches alphanumériques du clavier sont traitées par l'appareil comme étant relatives soit à la composition d'un repas, soit à la glycémie (dosage du glucose), soit au traitement médical prescrit, de manière à fournir les instructions correspondantes.

L'appareil portatif comporte avantageusement des moyens de branchement sur un micro-ordinateur ou analogue permettant de transférer les informations stockées dans la mémoire vive de l'appareil au micro-ordinateur en vue de leur analyse, la mémoire vive étant ré-initialisée après transfert de ces informations, la durée de stockage des informations dans l'appareil portatif étant supérieure à l'intervalle de temps moyen qui sépare deux branchements de l'appareil sur le micro-ordinateur. En retour le micro-ordinateur peut introduire des instructions et/ou des données dans la mémoire vive de l'appareil portatif.

Les moyens de calcul comprennent un microprocesseur.

L'invention sera mieux comprise à l'aide de la description qui suit d'un mode de réalisation particulier, avec référence aux dessins annexés.

La figure 1 de ces dessins est une vue en plan d'un appareil selon l'invention.

La figure 2 est une vue de gauche par rapport à la figure 1.

La figure 3, enfin, est un schéma synoptique de l'appareil.

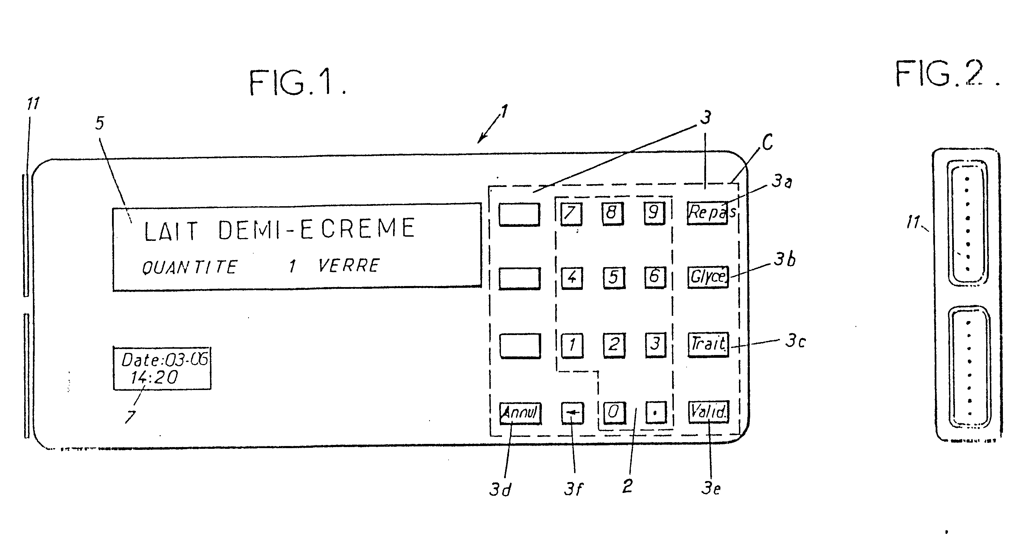

En se reportant à la figure 1, on peut voir un appareil portatif 1, du genre calculette, pour la saisie et le traitement d'informations relatives à la diététique et/ou à la santé d'une personne. En particulier l'exemple d'appareil 1 représenté est destiné à des personnes diabétiques soumises à un traitement et à un régime déterminé, ou, d'une manière plus générale, à des personnes suivant un régime diététique, notamment suivi par un organisme du genre «weight-watchers».

L'appareil comprend des moyens d'entrée des informations relatives à la santé, formés par un clavier C avec une série de touches 2, alphanumériques ou même numériques, et une série 3 de touches spécialisées. La partie alphanumérique 2 du clavier C représenté sur la figure 1 comporte essentiellement des chiffres car, dans l'exemple d'appareil illustré, les informations sont introduites par un code chiffré. Un lexique (non représenté) peut être prévu sur le dos de l'appareil pour établir la correspondance entre les informations en clair et les chiffres du code, l'appareil affichant de toute manière le libellé en clair correspondant au code introduit, comme indiqué ci-après.

Trois touches spécialisées 3a, 3b et 3c sont prévues pour faire traiter, par l'appareil 1, des informations relatives à diverses opérations, soit aux repas (touche 3a), soit à la glycémie, c'est-à-dire à la teneur du sang en glucose (touche 3b), soit à un traitement médical (touche 3c).

L'appareil est agencé de telle sorte que, lors de la frappe de l'une de ces touches spécialisées 3a, 3b, 3c, il est mis sous tension et son fonctionnement est prévu pour traiter des informations relatives à l'opération correspondant à la touche frappée.

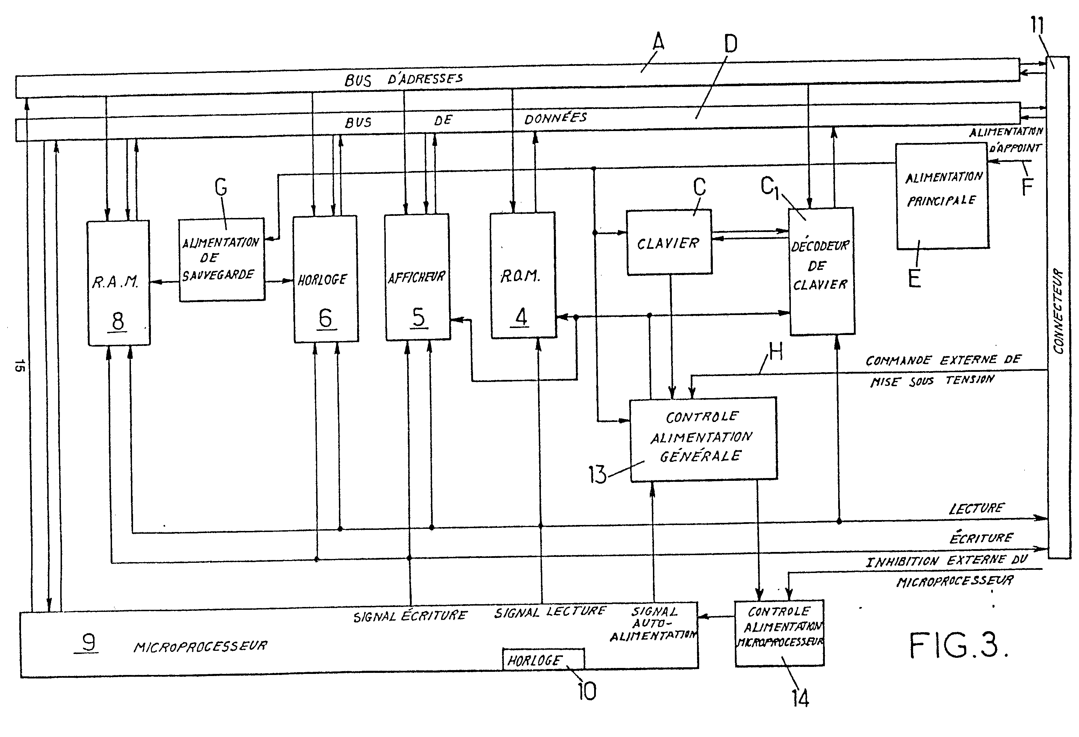

Une mémoire morte (ROM), schématiquement représentée sur la figure 3, est prévue et contient diverses instructions et données, parmi lesquelles une table des aliments (avec un code à huit bits on peut stocker en mémoire 256 aliments et boissons différents), avec la composition moyenne en protides, lipides, glucides et alcool de chaque aliment du boisson répertorié; d'autres instructions et données, explicitées plus loin, sont également stockées dans la mémoire morte 4.

Un écran 5 (figure 1) est prévu pour l'affichage.

L'appareil 1 comprend en outre des moyens d'horloge 6 (voir figure 3), notamment formés par une base de temps permettant de dater chaque entrée d'information; ces moyens d'horloge donnent la date, le jour, les heures et les minutes ou bien sont gradués en quarts d'heure (24 × 4 = 96 graduations par jour); le cas échéant, on peut prévoir un affichage permanent extérieur, représenté dans un rectangle 7 sur la figure 1, de l'heure donnée par les moyens d'horloge 6.

Une mémoire vive (RAM) 8 (figure 3), de capacité suffisante, est prévue pour le stockage des informations entrées sur le clavier C et des dates correspondantes, pendant un délai prédéterminé, par exemple de 45 jours. Cette mémoire 8 est également destinée à stocker des portions évolutives de programme correspondant à des données particulières à l'usager.

Des moyens de calcul 9 (figure 3), avantageusement formés par un microprocesseur, sont prévus pour déterminer, à partir des informations entrées sur la clavier C et des instructions et données stockées dans la mémoire morte 4, des instructions destinées à l'usager de l'appareil relatives à la diététique et/ou à la santé de l'usager, ces instructions apparaissant sur l'écran d'affichage 5.

La mémoire morte 4 contient, en plus des données fixes, notamment celles relatives aux aliments, le programme moniteur commandant le microprocesseur 9.

Les données circulent par un faisceau de lignes «bus de données» D, tandis que les adresses circulent par un faisceau de lignes «bus d'adresses» A.

L'appareil 1 est, en outre, équipé d'un connecteur 11 (figures 1, 2 et 3) pour liaison avec un interface: appareil 1/ordinateur (en particulier micro-ordinateur) et éventuellement d'un connecteur (non représenté) pour liaison avec un analyseur glycémique.

Le microprocesseur 9 est muni de sa propre horloge à quartz 10 qui permet le déroulement des opérations, mais qui n'a pas de fonction de datage de l'entrée des informations, fonction qui est assurée par l'horloge 6.

Pour faciliter la saisie des informations, on prévoit, dans la série 3 de touches spécialisées, en plus des touches 3a, 3b, 3c mentionnées ci-dessus,

– une touche d'«annulation» 3d permettant d'annuler les informations que l'on vient de frapper sur le clavier C et qui apparaissent sur l'écran 5,

– une touche de «validation» 3e permettant, après contrôle visuel (sur l'écran 5) des informations frappées, de valider ces informations et de les faire entrer dans la mémoire vive 8, et

– une touche 3f, représentée munie d'une flèche sur la figure 1, permettant d'effectuer des corrections. Au clavier C est associé un décodeur de clavier C1.

Les moyens d'horloge interne 6 permettent, non seulement de dater chaque information recueillie, mais rendent également possible la fonction d'aide-mémoire, par exemple en assurant, à l'aide d'une alarme sonore ou visuelle (clignotant), le rappel de l'horaire d'un traitement, ou d'un contrôle glycémique pour un diabétique, de la date du rendez-vous de consultation chez le médecin ou un organisme de surveillance (weight-watcher), etc.

La mémoire morte 4 contient le programme moniteur assurant les fonctions générales de la calculette: gestion du clavier, gestion de l'affichage, éventuellement affichage continu de l'heure, surveillance de la place disponible dans la mémoire vive 8, gestion des périphériques, etc.

La mémoire morte 4 comprend en outre des programmes correspondant aux trois touches spécialisées 3a (repas), 3b (glycémie) et 3c (traitement).

1. En cas d'actionnement de la touche «repas» 3a, la calculette va fonctionner suivant le programme «diététique» de la mémoire morte 4. Lors de la frappe au clavier du code chiffré ou mnémonique d'un aliment, le nom de celui-ci (par exemple lait demi-écrémé, comme indiqué sur la figure 1), éventuellement en abrégé, apparaît affiché en clair sur l'écran 5. Le programme de la mémoire morte 4 réclame alors la quantité absorbée, dans l'unité de mesure courante, qui peut être, par exemple, de manière très pratique, la cuiller à soupe ou à café ou le verre (comme indiqué par exemple sur la figure 1). Chaque aliment absorbé, référencé par un code interne sur huit bits (possibilités de cataloguer 256 aliments) est mis en mémoire, par la mémoire vive 8, sous cette forme avec la quantité et la date.

Ainsi, au moment du repas, la personne concernée enregistre la composition de celui-ci et en obtient la quantification protidique, lipidique, glucidique, alcoolique et calorique totale qui apparaît sur l'écran 5. Plusieurs essais de composition de repas peuvent être effectués, avant de valider, en frappant sur la touche 3e, la composition du repas et avant de consommer ce repas.

Cumulés sur 24 heures, ces résultats permettent d'équilibrer les rations alimentaires journalières. Après chaque repas, le crédit restant en protides, lipides, glucides, alcool et calories est déterminé automatiquement par la calculette en déduisant du crédit précédent la composition du repas qui vient d'être mise en mémoire. Lors de la frappe sur la touche «repas» 3a, le crédit actuel en protides, lipides, glucides et calories apparaît sur l'écran 5.

Chaque jour, c'est-à-dire toutes les 24 heures, à une heure déterminée donnée par l'horloge interne 6, par exemple à minuit, le crédit alimentaire quotidien est remis à sa valeur initiale, valeur qui aura été prescrite par le médecin ou un organisme de surveillance, par exemple lors d'une consultation ou une réunion précédente, et introduite dans la mémoire vive 8 grâce au connecteur 11.

Le calcul automatique, par l'appareil 1, des protides, lipides, glucides, alcool et calories d'un repas, est effectué grâce à la table stockée dans la mémoire morte 4, où chaque aliment ou boisson comporte sa composition moyenne et approchée en protides, lipides, glucides, alcool et calories.

On peut obtenir à l'affichage, sur l'écran 5, le bilan pour chaque aliment et annuler une erreur de codage à tout moment.

La sommation est effectuée pour la totalité du repas, et la différence en plus ou en moins par rapport au régime théorique, comme expliqué précédemment, reste connue jusqu'au repas suivant. La composition complète du repas, associée à l'horaire fourni par l'horloge 6, est stockée en mémoire vive 8.

Il y a lieu de signaler, en ce qui concerne la surveillance de l'alimentation, qu'il existe certaines associations ou organismes privés ou publics, extra-médicaux, qui tentent de venir en aide aux personnes présentant des troubles de l'alimentation, des obésités, etc.

Un exemple, originaire des Etats-Unis, s'est développé dans de nombreux pays; il s'agit de l'association des «weight-watchers» (littéralement: «ceux qui surveillent leur poids»), qui a fait de nombreux adeptes.

Ces associations mettent entre les mains de leurs adhérents le maximum de moyens pour les aider à lutter contre les erreurs diététiques, et des réunions périodiques sont organisées pour analyser les progrès de chacun et les erreurs alimentaires éventuelles.

La calculette selon l'invention, dotée de moyens de calcul et de mémoire convenables, permet d'évaluer avec précision la quantité de lipides, protides, glucides, alcool et calories de chaque repas, introduite plat par plat grâce au clavier.

La RAM de la calculette permet de mettre en mémoire ces informations, et, lors d'une réunion périodique, une analyse systématique pour chaque participant peut être établie par l'ordinateur de l'association, grâce au connecteur 11 de la calculette.

Ces informations étant mémorisées avec la date et l'heure lors de l'introduction au clavier, on peut reconstituer même la «chronologie diététique», un grand nombre de troubles de l'alimentation étant lié à des problèmes d'horaires de consommation (collations, grignotages, sauts de repas).

2. L'actionnement de la touche «glycémie» 3b fait intervenir le programme correspondant stocké dans la mémoire morte 4. La valeur de la glycémie (teneur du sang en glucose) peut être introduite manuellement, par la personne conernée, qui frappe sur le clavier C la valeur qu'elle a déterminée à l'aide d'un moyen de dosage rapide dont elle dispose. Selon une autre possibilité, cette valeur de la glycémie peut être introduite automatiquement, à l'aide d'un analyseur glycémique qui serait branché sur un connecteur (en plus du connecteur 11) non représenté, prévu sur la calculette. La valeur de la glycémie apparaît sur l'écran 5 de la calculette, qu'elle soit introduite manuellement ou automatiquement. La personne concernée valide la valeur affichée, en appuyant sur la touche «validation» 3e. Le résultat de la mesure, ainsi que l'horaire fourni par l'horloge interne 6, sont alors stockés dans la mémoire vive 8.

Comme indiqué précédemment, l'appareil 1 peut assurer un rappel automatique de l'horaire du contrôle glycémique.

3. Dans le cas d'une personne soumise à un traitement médical, par exemple dans le cas d'un diabétique, l'appareil 1 peut également rappeler, à l'aide d'une alarme sonore ou visuelle, l'horaire de prise de médicaments antidiabétiques ou de l'injection d'insuline. L'utilisateur, après avoir frappé sur la touche «traitement» 3c, peut enregistrer le type de traitement et la dose de médicament absorbé ou d'insuline injectée. Ces informations sont stockées dans la mémoire vive 8 ainsi que l'heure et la date auxquelles ce traitement a été effectué.

D'une manière générale, les fonctions annexes, telles que le rappel de la date de la prochaine consultation, l'affichage intermittent d'une information particulière, peuvent être assurés par un programme spécifique utilisant la mémoire vive 8.

Toutes les informations introduites (composition des repas, valeur glycémique, type et dose de traitement), assorties des horaires correspondants, sont donc stockées dans la mémoire vive 8 de la calculette. L'ensemble est agencé de manière à suspendre automatiquement le fonctionnement, sans perte d'informations, si la capacité de stockage de la mémoire 8 est dépassée.

Lors de la consultation suivante, par branchement sur le connecteur 11, par l'intermédiaire d'un interface approprié, toutes les informations stockées dans la mémoire vive 8 sont transférées dans l'ordinateur du médecin qui peut réaliser un traitement des données instantanément ou en différé.

Compte-tenu d'une fréquence mensuelle de consultations, on prévoit une possibilité de stockage pour une période de 45 jours minimum, pour tenir compte des décalages possibles de rendez-vous.

A chaque consultation médicale, les indications stockées dans la mémoire vive 8, relatives aux aliments, à la glycémie et aux prises de médicaments, sont effacées après avoir été transférées dans l'ordinateur du médecin, de telle sorte que la mémoire vive 8 soit prête pour de nouveaux enregistrements.

Il est à noter que l'appareil 1 est «personnalisé» dès sa première mise en service, lors de la première consultation. Cette personnalisation consiste à introduire dans la mémoire vive 8 des renseignements relatifs à la personne cocernée, en particulier: nom de cette personne, caractéristiques du régime alimentaire, des médicaments et des traitements prescrits, date de la consultation en cours et de la prochaine consultation, etc.

Ces informations peuvent être mises à jour par le médecin lors des consultations ultérieures.

La base de temps 6 peut être agencée de telle sorte que sa sortie permette de mémoriser les dates, quart d'heure par quart d'heure, à partir d'une date «zéro» entrée en mémoire depuis l'ordinateur du médecin, cette date «zéro» étant enregistrée à chaque rendez-vous. Pour une période de 45 jours, les intervalles de temps d'un quart d'heure représentent donc environ 4500 périodes pouvant être mémorisées. A chaque consultation, la date exacte est corrigée en même temps qu'est donné le départ du comptage de temps.

L'écran d'affichage 5 comporte, en plus des repères de signalisation, au moins une ligne de 30 caractères alphanumériques pour l'inscription en clair des aliments et de la quantité absorbée, et plus généralement de toute information frappée au clavier et toute instruction donnée par l'appareil.

De préférence, la mémoire morte 4 et la mémoire vive 8 ont une capacité de 8 K Bytes chacune.

L'appareil 1 est équipé d'un double système d'alimentation par batteries rechargeables E, lui permettant un fonctionnement autonome. L'appareil 1 peut, le cas échéant, être prévu pour fonctionner également sur une prise de courant F. On prévoit avantageusement une possibilité de tester l'état de charge des batteries et de contrôler l'alimentation générale en 13 et l'alimentation du microprocesseur en 14.

La mémoire vive 8 reste alimentée, à partir d'une seconde batterie G (alimentation de sauvegarde), même en cas de non-utilisation de la calculette, et même lorsque la batterie principale E est déchargée.

Pour le dosage du glucose sanguin, on peut utiliser un appareil automatique à affichage digital comme il en existe sur le marché, par exemple celui connu sous la dénomination «glucometer AMES».

Le connecteur 11 de l'interface calculette/ordinateur est prévu pour s'adapter aux divers ordinateurs de cabinets médicaux généralement employés. Il est compatible avec les standards de transmission en micro-informatique.

Le contrôle de la mise sous tension et du partage des bus par l'interface est réalisé avantageusement comme suit.

Un premier circuit électrique, alimenté en permanence par la batterie E de l'appareil, reçoit des informations de contrôle:

– du clavier C lui-même alimenté en permanence
– du microprocesseur 9
– du connecteur d'interface 11.

A – En utilisation portative, le déclenchement du clavier C active ce circuit, lequel draine alors le courant vers l'ensemble des composants, sauf le microprocesseur 9. Parmi ces composants, un second circuit de contrôle est alimenté en courant à partir de cet instant. Ce second circuit reçoit une information de contrôle supplémentaire du connecteur 11 d'interface, en fonction de laquelle il conduit ou non le courant vers le microprocesseur 9.

1er cas: l'information est absente; alors le second circuit est conducteur et le microprocesseur 9 se met en fonctionnement; le programme interne se déroule et commande alors un signal qui retourne activer de façon permanente le premier circuit, de sorte que celui-ci reste conducteur: c'est «l'auto-alimentation»; dès lors, le clavier C peut être relâché, le microprocesseur poursuit son activité, jusqu'au moment où il coupera son signal d'auto-alimentation.

2ème cas: l'information est présente; alors le second circuit reste non conducteur et le microprocesseur 9 ne se met pas en fonctionnement; il y a coupure de l'ensemble de l'alimentation dès que le clavier C est relâché.

B – En utilisation via le connecteur 11, l'interface peut jouer le rôle du clavier en activant le premier circuit. Si elle ne donne pas l'information décrite plus haut, le microprocesseur 9 s'active (voir 1er cas ci-dessus).

Par contre si l'interface donne cette information, le microprocesseur 9 ne s'active pas, alors que tous les autres composants sont sous tension et fonctionnels. L'interface, par l'intermédiaire du connecteur 11, disposant de la ligne H de contrôle, du bus de données D et de celui des adresses A, est par conséquent en mesure d'exploiter toutes les ressources de la calculette, sans interférence avec les signaux du microprocesseur 9 de celle-ci qu'il inhibe.

Il est clair que la calculette 1 de l'invention peut être utilisée et adaptée à d'autres types d'informations relatives à l'alimentation et/ou à la santé d'une personne. Par exemple un tel appareil pourrait être adapté aux informations relatives à la tension artérielle, au rythme cardiaque.

Comme exemples, non limitatifs, de circuits utilisés dans l'appareil, on peut donner les références suivantes:

| | |
|---|---|
| mémoire morte 4: | référence 23C64 Hughes |
| mémoire vive 8: | référence TMM 20C64 Toshiba |
| microprocesseur 9: | référence 80C51 marque Intel |
| écran d'affichage 5: | EPSON EA-Y 20 025 AZ |
| horloge 6: | ICM 7223 IPL Intersil |

L'organigramme donné ci-après permet de mieux comprendre le fonctionnement de l'appareil suivant le programme «repas».

**Mise en route**

```
                    ┌─────────────────────────┐
                    │     Appareil arrêté      │
                    └────────────┬────────────┘
                                 │
                         Pression touche repas
                                 │
                    ┌─────────────────────────┐
                    │   Mise en fonctionnement │
                    ├─────────────────────────┤
                    │   Routine de contrôle    │
                    │    de mise en route      │
                    └────────────┬────────────┘
```

Y'a t-il un repas en attente de validation? → oui → Routine de validation de repas en attente

non

oui ← Jour actuel est-il égal à jour précédent?

non

Copier crédits imposés dans crédits du jour

Heure + date + (info = R) en mémoire

**Affichage Crédits Actuels**

Affichage alternatif CREDIT ACTUEL PROTIDES/ LIPIDES/GLUCIDES/CALORIES

Affichage CODE ALIMENT OU ARRET (TOUCHE ANNULATION) pendant 10 sec

oui ← Attente totale ≤ 1 mn? sans pression de touche

non

Pression touche numérique? → oui

non

Pression touche ANNULATION → oui

non

oui ← Autre touche → non → Extinction – Arrêt

**Introduction code de l'aliment**

Routine d'introduction nombre entier 3 digits

Contrôle de cohérence

Affichage libellé correspondant; ex: LAIT DEMIECREME Qté VERRES

Pression touche numérique? — oui

non

Pression touche ANNULATION? — oui

non

Attente totale ≥ 1 mn? — oui

non

**Introduction quantité de l'aliment**

Routine d'introduction nombre décimal 4 digits, point compris

Contrôle de cohérence

**Affichage bilan pour cet aliment**

Affichage alternatif des équivalents en PROTIDES/LIPIDES/GLUCIDES/CALORIES

Affichage CONSERVER (VALID) SUPPRIMER (ANNUL) pendant 10 sec.

**Correction possible**

Pression touche ANNULATION? — oui

non

non — Pression touche VALIDATION? ou attente ≥ 1 mn

oui

Mise en mémoire

Calcul du cumul du repas

**Affichage bilan depuis début repas**

Affichage alternatif du cumul du repas en PROTIDES/LIPIDES/GLUCIDES/CALORIES

Affichage NOUVEAU CODE ALIMENT ou FIN REPAS (VALID)

**Aliment suivant**

oui — Touche numérique?

non

**Fin de repas**

non — Validation ou attente ≥ 1 mn?

oui

Calcul crédits fin de repas

**Affichage crédit fin de repas**

Affichage alternatif du crédit fin de repas en PROTIDES/LIPIDES/GLUCIDES/CALORIES

Affichage REPAS ACCEPTE (VALID) ou REFUS DU REPAS (ANNUL) pendant 10 sec.

**Ajout d'un aliment oublié**

**Non enregistrement du repas**

**Enregistrement du repas**

**Repas restant à valider**

**Arrêt automatique**

Touche numérique? — oui / non

Touche ANNULATION? — non / oui

Affichage REPAS NON MEMORISE

Touche VALIDATION? — non / oui

Copier crédits fin repas dans crédits du jour

Mise à jour des pointeurs mémoire

Affichage REPAS MEMORISE

Attente ⩾ 2 mn — non / oui

Positionner l'indicateur de Repas en attente

Affichage REPAS RESTANT A VALIDER

Clignotement de l'affichage pendant 10 sec.

Extinction – Arrêt

## Revendications

1. Appareil portatif de saisie et de traitement d'informations relatives à la diététique et/ou à la santé d'une personne, du type comportant, une série (2) de touches alpha-numériques pour l'entrée d'informations relatives à la diététique et/ou à la santé de l'usager; au moins une mémoire morte (4) dans laquelle sont stockées des données et des instructions en relation avec ces informations; des moyens d'affichage (5) des informations introduites et des renseignements les concernant en provenance de la mémoire morte; des moyens d'entrée effective (3e) et de refus d'entrée (3d) de ces informations; des moyens de calcul (9) propres à déterminer, à partir des informations entrées et des données stockées dans la mémoire morte, des instructions destinées à l'usager de l'appareil et relatives à la diététique et/ou à la santé de l'usager; des moyens d'alimentation électrique principale (E) de l'appareil; et caractérisé en ce qu'il comporte également des moyens d'horloge (6) permettant de dater chaque entrée d'information; au moins une mémoire vive (8) propre à stocker les informations entrées ainsi que leur date et des moyens de communication (11) pour permettre le transfert des informations stockées dans la mémoire vive vers une unité de traitement d'informations extérieure ainsi qu'en retour l'introduction, dans cette mémoire vive, d'instructions en provenance de ladite unité.

2. Appareil selon la revendication 1, destiné à des personnes soumises à un régime alimentaire, caractérisé par le fait que la mémoire morte (4) contient des données relatives à un certain nombre d'aliments, ces données étant notamment constituées par la teneur moyenne en protides, lipides, glucides, alcool et calories des aliments, les moyens de calcul (9) étant agencés pour déterminer, pour chaque aliment, en fonction de la quantité qui sera absorbée, la quantité de protides, lipides, glucides, alcool et calories, ces informations étant ensuite stockées, avec la date, dans la mémoire vive (8).

3. Appareil selon la revendication 1 ou 2, caractérisé par le fait que la mémoire vive (8) contient des informations, notamment inscrites lors d'une consultation médicale, qui concernent un régime ou traitement quotidien, notamment les quantités de référence quotidienne de protides, lipides, glucides, alcool, calories pour la personne intéressée, et que les moyens de calcul (9) font apparaître, avant chaque entrée d'information, le crédit disponible pour la journée en cours, les moyens de calcul (9) modifiant au fur et à mesure le crédit en fonction des informations introduites et les moyens d'horloge (6) commandant la remise du crédit disponible à sa valeur de référence quotidienne, à une heure déterminée, pour la journée suivante.

4. Appareil selon l'une quelconque des revendications précédentes, caractérisé par le fait qu'il comporte des touches spécialisées (3a, 3b, 3c) pour traiter des informations relatives à diverses opérations, notamment aux repas (touche 3a), à la glycémie (touche 3b), à un traitement médical (touche 3c), l'actionnement d'une de ces touches spécialisées sélectionnant le traitement des informations relatives à l'opération correspondant à la touche spécialisée.

5. Appareil selon l'une quelconque des revendications précédentes, caractérisé par le fait qu'il comprend des moyens de branchement sur un appareil de dosage automatique du glucose sanguin.

6. Appareil selon l'une quelconque des revendications précédentes, caractérisé par le fait que la mémoire morte (4) et la mémoire vive (8) ont une capacité de 8 K bytes.

7. Appareil selon l'une quelconque des revendications précédentes, caractérisé par le fait qu'il comporte des moyens d'alimentation électrique de secours (G) pour que la mémoire vive (8) reste alimentée même en cas de non utilisation ou lorsque les moyens d'alimentation électrique principale (E) sont déchargés.

8. Appareil selon l'une quelconque des revendications précédentes, caractérisé en ce qu'il comporte des moyens pour suspendre automatiquement le fonctionnement de l'appareil, sans pertes d'informations, si la capacité de stockage de la mémoire vive (8) est dépassée.

**Claims**

1. Portable apparatus for the acquisition and processing of information relative to the diet and/or health of a person, of the type comprising a series (2) of alphanumeric keys for the input of information relative to the diet and/or health of the user; at least one read-only memory (4) in which are stored data and instructions in relation with this information; means (5) for displaying the information introduced and the information concerning it coming from the read-only memory; means for effective input (3e) and means for input refusal (3d) of this information; calculating means (9) adapted to determine, from the entered information and the data stored in the read-only memory, instructions intended for the user of the apparatus and relative to the diet and/or health of the user; principal electrical supply means (E) for the apparatus; and characterized in that it also comprises clock means (6) for dating every input of information; at least one read-write memory (8) adapted to store the entered information as well as its date and communication means (11) for allowing the transfer of the information stored in the read-write memory towards an outside information processing unit and, in return, the introduction into this read-write memory, of instructions coming from said unit.

2. Apparatus according to Claim 1, intended for persons subjected to a food diet, characterized in that the read-only memory (4) contains data relative to a certain number of food-stuffs, such data being in particular constituted by the average content of protids, lipids, carbohydrates, alcohol and calories of the foodstuffs, the calculating means (9) being arranged to determine, for each foodstuff, as a function of the quantity which will be absorbed, the quantity of protids, lipids, carbohydrates, alcohol and calories, this information then being stored, with the date, in the read-write memory (8).

3. Apparatus according to Claim 1 or 2, characterized in that the read-write memory (8) contains information, in particular recorded during a medical consultation, which concerns a daily diet or treatment, particularly the daily reference quantities of protids, lipids, carbohydrates, alcohol, calories for the person in question, and the calculating means (9) show, before every input of information, the credit available for the day under way, the calculating means (9) modifying the credit accordingly as a function of the information introduced and the clock means (6) controlling the return of the available credit to its daily reference value at a determined time, for the following day.

4. Apparatus according to any one of the preceding Claims, characterized in that it comprises specialist keys (3a, 3b, 3c) for processing information relative to various operations, particularly to meals (key 3a), glycemia (key 3b), to a medical treatment (key 3c), the actuation of one of these specialist keys selecting the processing of the information relative to the operation corresponding to the specialist key.

5. Apparatus according to any one of the preceding Claims, characterized in that it comprises means for connection onto an apparatus for automatically dosing blood glucose.

6. Apparatus according to any one of the preceding Claims, characterized in that the read-only memory (4) and the read-write memory (8) have a capacity of 8 K bytes.

7. Apparatus according to any one of the preceding Claims, characterized in that it comprises emergency electrical supply means (G) for the read-write memory (8) to remain supplied even in the event of non-use or when the principal electrical supply means (E) have discharged.

8. Apparatus according to any one of the preceding Claims, characterized in that it comprises means for automatically suspending operation of the apparatus, without loss of information, if the storage capacity of the read-write memory (8) is exceeded.

**Patentansprüche**

1. Tragbare Vorrichtung zum Erfassen und Verarbeiten von Informationen über die Diätetik und/oder Gesundheit einer Person mit einer Reihe (2) von alphanumerischen Tasten für die Eingabe von Informationen über die Diätetik und/oder Gesundheit des Benutzers; mit mindestens einem Lesespeicher (4), in dem Daten und Anweisungen bezüglich dieser Informationen gespeichert sind; mit Anzeigemitteln (5) für die angegebenen Informationen und für sie betreffende, aus dem Lesespeicher stammende Aussagen; mit Mitteln zur effektiven Eingabe (3e) und zur Sperrung der Eingabe (3d) dieser Informationen; mit Mitteln zum Berechnen (9), die aus den eingegebenen Informationen und den im Lesespeicher gespeicherten Daten Anweisungen, die sich auf die Diätetik und/oder Gesundheit des Benutzers beziehen, für den Benutzer der Vorrichtung bestimmen können; und mit Mitteln zur elektrischen Hauptversorgung (E) der Vorrichtung, dadurch gekennzeichnet, dass sie auch Mittel zur Zeiterfassung (6) zum Datieren jeder Informationseingabe, dass sie einen Schreib/Lese-Speicher (8) zum Speichern der eingegebenen Informationen wie auch ihres Datums und dass sie Kommunikationsmittel (11) zum Übertragen der im Schreib/Lese-Speicher gespeicherten Daten an eine externe Einheit zum Verarbeiten der Informationen wie auch in der anderen Richtung zum Eingeben von Informationen aus dieser Einheit in den Schreib/Lese-Speicher umfasst.

2. Vorrichtung nach Anspruch 1 für sich einer Diät unterziehenden Person, dadurch gekennzeichnet, dass der Lesespeicher (4) Daten über eine bestimmte Anzahl von Nahrungsmitteln enthält, wobei diese Daten insbesondere aus dem mittleren Gehalt der Nahrungsmittel an Protiden, Lipiden, Zucker, Alkohol und Kalorien bestehen, wobei die Mittel zum Berechnen (9) so beschaffen sind, dass sie für jedes Lebensmittel in Funktion von der aufgenommenen Menge die Menge an Protiden, Lipiden, Zucker, Alkohol und Kalorien bestimmen, wobei diese Informationen anschliessend mit dem Datum in Schreib/Lese-Speicher (8) gespeichert werden.

3. Vorrichtung nach Anspruch 1 oder 2, dadurch gekennzeichnet, dass der Schreib/Lese-Speicher (8) Informationen enthält, die insbesondere bei einer medizinischen Behandlung eingeschrieben worden sind und die eine tägliche Diät oder Behandlung betreffen, insbesondere die täglichen Bezugsmengen an Protiden, Lipiden, Zucker, Alkohol, Kalorien für die betreffende Person, und dass die Mittel zum Berechnen (9) vor jeder Eingabe von Informationen das verfügbare Guthaben für den laufenden Tag anzeigen lassen, wobei die Mittel zum Berechnen (9) das Guthaben in Funktion der eingegebenen Informationen ändern und die Mittel zur Zeiterfassung (6) das Rückstellen des verfügbaren Guthabens auf seinen täglichen Bezugswert für den folgenden Tag zu einer bestimmten Stunde steuern.

4. Vorrichtung nach einem der vorhergehenden Ansprüche, dadurch gekennzeichnet, dass sie Sondertasten (3a, 3b, 3c) zum Verarbeiten von Informationen über diverse Vorgänge umfasst, insbesondere über Mahlzeiten (3a), Glykämie (3b), medizinische Behandlung (3c), wobei das Betätigen einer dieser Sondertasten die Verarbeitung der Informationen über den der Sondertaste entsprechenden Vorgang anwählt.

5. Vorrichtung nach einem der vorhergehenden Ansprüche, dadurch gekennzeichnet, dass sie Mittel zum Verzweigen auf ein automatisches Dosiergerät für Blutglucose umfasst.

6. Vorrichtung nach einem der vorhergehenden Ansprüche, dadurch gekennzeichnet, dass der Lesespeicher (4) und der Schreib/Lese-Speicher (8) eine Kapazität von 8 kByte haben.

7. Vorrichtung nach einem der vorhergehenden Ansprüche, dadurch gekennzeichnet, dass sie Mittel zur elektrischen Notstromversorgung (G) umfasst, damit der Schreib/Lese-Speicher (8) auch bei ausgeschaltetem Gerät oder bei Ausfall der elektrischen Hauptversorgung (E) versorgt bleibt.

8. Vorrichtung nach einem der vorhergehenden Ansprüche, dadurch gekennzeichnet, dass sie Mittel zum automatischen Abbrechen der Funktion der Vorrichtung umfasst, ohne Informationsverlust, wenn die Speicherkapazität des Schreib/Lese-Speichers (8) überschritten wird.

FIG.1.

FIG.2.

LAIT DEMI-ECREME

QUANTITE    1  VERRE

Date:03-06
14:20

7  8  9  Repas  3a
4  5  6  Glyce  3b
1  2  3  Trait  3c
Annul  ←  0  ·  Valid.

3d  3f  2  3e

EP 0 128 054 B1

FIG.3.